(19) Europäisches Patentamt
European Patent Office
Office européen des brevets

(11) **EP 2 608 848 B1**

(12) **EUROPÄISCHE PATENTSCHRIFT**

(45) Veröffentlichungstag und Bekanntmachung des Hinweises auf die Patenterteilung:
**19.04.2017 Patentblatt 2017/16**

(21) Anmeldenummer: **11725041.5**

(22) Anmeldetag: **06.06.2011**

(51) Int Cl.:
*A61Q 5/08* (2006.01)    *A61K 8/22* (2006.01)
*A61K 8/23* (2006.01)    *A61K 8/44* (2006.01)
*A61K 8/46* (2006.01)

(86) Internationale Anmeldenummer:
**PCT/EP2011/059257**

(87) Internationale Veröffentlichungsnummer:
**WO 2012/025269 (01.03.2012 Gazette 2012/09)**

(54) **BLONDIERUNG MIT REDUKTIVER VORBEHANDLUNG**

BLEACHING WITH REDUCTIVE PRE-TREATMENT

DÉCOLORATION AVEC UN PRÉTRAITEMENT RÉDUCTEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.08.2010 DE 102010039878**

(43) Veröffentlichungstag der Anmeldung:
**03.07.2013 Patentblatt 2013/27**

(73) Patentinhaber: **Henkel AG & Co. KGaA 40589 Düsseldorf (DE)**

(72) Erfinder:
• **GROß, Wibke**
  **41836 Hückelhoven (DE)**
• **KROOS, Astrid**
  **40789 Monheim (DE)**
• **JANßEN, Frank**
  **41470 Neuss (DE)**
• **KLEEN, Astrid**
  **20457 Hamburg (DE)**
• **KNÜBEL, Georg**
  **40219 Düsseldorf (DE)**

(56) Entgegenhaltungen:
**EP-A1- 1 023 891      EP-A1- 2 098 218
EP-A1- 2 295 029      WO-A1-03/082236
DE-A1- 1 617 829      DE-A1- 2 307 596
FR-A1- 2 719 472      US-A- 3 823 231**

Bemerkungen:
Die Akte enthält technische Angaben, die nach dem Eingang der Anmeldung eingereicht wurden und die nicht in dieser Patentschrift enthalten sind.

**Beschreibung**

Gegenstand der vorliegenden Anmeldung sind Mehrkomponentenverpackungseinheiten zur verbesserten Aufhellung und/oder Blondierung von keratinischen Fasern, insbesondere menschlichen Haaren. Weiterhin werden ein- und mehrstufige Verfahren zur Anwendung dieser Mehrkomponentenverpackungseinheiten in der Behandlung von keratinischen Fasern beschrieben.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Für diesen Zweck sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxodisulfatsalzen und/oder Peroxomonosulfatsalzen eingesetzt. Zur Intensivierung der Blondierwirkung enthalten die Mittel höhere Einsatzkonzentrationen an Wasserstoffperoxid und Persulfaten.

Mit der Aufhellung geht jedoch auch eine Schädigung des Haares einher, da nicht nur die Farbstoffe des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares bis hin zu Haarbruch. Je größer die Menge des eingesetzten Wasserstoffperoxids und gegebenenfalls der Peroxodisulfate ist, desto stärkere Schädigungen werden daher in der Regel auf der Keratinfaser hervorgerufen.

Infolge der hiermit verbundenen Erhöhung der Haarschädigung einerseits sowie gesetzlichen und toxikologischen Vorgaben andererseits kann die Konzentration dieser Oxidationsmittel jedoch nicht beliebig erhöht werden, sondern unterliegt vorgegebenen Grenzen. Auch bei Verwendung der maximal möglichen Konzentrationen an Oxidationsmitteln können Haare nur in beschränktem Ausmaß blondiert werden. Insbesondere dunkles, dunkelbraunes oder schwarzes Haar lässt sich in einem Schritt maximal um 4 bis 6 Nuancen aufhellen, wodurch bei dieser Ausgangshaarfarbe das Erreichen eines hellen Blondtons nicht möglich ist.

Es war daher die Aufgabe der vorliegenden Erfindung, neuartige Methoden zum Aufhellen bzw. Blondieren von Haaren bereitzustellen, welche in ihrer Aufhellleistung den üblichen auf dem Markt befindlichen Mitteln überlegen sind, gleichzeitig jedoch keine verstärkte Haarschädigung aufweisen.

Aus der Offenbarung der DE 1617829 sind Blondiermittel bekannt, die neben Wasserstoffperoxid und Persulfaten bestimmte organische schwefelhaltige Verbindungen enthalten. Aus US 2201929 sind Dauerwellmittel bekannt, bei denen zunächst ein Reduktionsmittel auf das Haar aufgetragen wird und anschließend eine Oxidation mit Wasserstoffperoxid oder Ammoniumpersulfat durchgeführt wird. Das Dokument FR 2719472A1 offenbart ein Blondierverfahren, bei dem das Haar zunächst mit Laserlicht oder - äquivalent - dreimal mit einer wässrigen Wasserstoffperoxid/Persulfat-Mischung aufgehellt, dann gewaschen und getrocknet, anschließend mit einer organischen Thiolverbindung (z. B. Thioglycolsäure) behandelt, wieder ausgespült und abschließend erneut mit Wasserstoffperoxid ohne die Anwesenheit von Persulfatsalzen behandelt wird.

Für den Fachmann war es daher nicht vorhersehbar, dass durch eine Vorbehandlung der Keratinfasern mit einem organischen Thiol vor dem eigentlichen Oxidationsschritt in Blondierverfahren die Aufhellleistung auf den keratinischen Fasern signifikant verbessert werden kann.

Ebenfalls nicht vorhersehbar für den Fachmann war, dass die zuvor beschriebene Vorbehandlung mit einem organischen Thiol nur die Bleichwirkung von Wasserstoffperoxid/Persulfat-Gemischen, jedoch nicht die Bleichwirkung von Wasserstoffperoxid allein zu verstärken vermag.

Ein erster Gegenstand der vorliegenden Erfindung ist daher ein Verfahren zur Aufhellung von keratinischen Fasern, insbesondere menschlichen Haaren, bei dem die Komponenten einer Mehrkomponentenverpackungseinheit (Kit-of-Parts) zur Aufhellung von keratinischen Fasern auf die keratinhaltigen Fasern aufgebracht, 5 bis 60 Minuten auf der Faser belassen und anschließend die Fasern mit Wasser gespült werden oder einem tensidhaltigen Reinigungsmittel ausgewaschen werden, wobei die Mehrkomponentenverpackungseinheit mindestens die folgenden drei getrennt voneinander konfektionierten Komponenten enthält:

   i) eine erste Komponente (I), die in einem kosmetisch akzeptablen Trägermedium mindestens eine organische Thiol-Verbindung enthält,
   ii) eine zweite Komponente (II), die mindestens ein Persulfatsalz enthält, und
   iii) eine dritte Komponente (III), die in einem kosmetisch akzeptablen Trägermedium Wasserstoffperoxid enthält,

wobei das Verfahren dadurch gekennzeichnet ist, dass

a) in einem ersten Schritt die erste Komponente (I) des Kits, enthaltend in einem kosmetisch akzeptablen Trägermedium mindestens eine organische Thiol-Verbindung, auf die trockenen oder feuchten Fasern aufgetragen wird und für eine Einwirkzeit von 5 bis 45 min auf den Fasern belassen wird,

b) in einem zweiten Schritt die keratinischen Fasern mit Wasser oder einem tensidhaltigen Reinigungsmittel gespült werden,

c) in einem weiteren Schritt eine Mischung aus den Komponenten (II) und (III) des Kits auf die Fasern aufgetragen wird und für eine Einwirkzeit von 10 bis 45 min auf den Fasern belassen wird,

d) und anschließend die Fasern mit Wasser gespült werden oder einem tensidhaltigen Reinigungsmittel ausgewaschen werden.

Unter keratinhaltigen bzw. keratinischen Fasern werden erfindungsgemäß Pelze, Wolle, Federn und insbesondere menschliche Haare verstanden. Obwohl die erfindungsgemäß verwendeten Mittel in erster Linie zum Blondieren und/oder Aufhellen von keratinhaltigen Fasern geeignet sind, steht prinzipiell einer Verwendung auch auf anderen Gebieten nichts entgegen.

Eine Mehrkomponentenverpackungseinheit umfasst mehrere Einzelkomponenten, die getrennt voneinander konfektioniert sind, sowie eine gemeinsame Verpackung für diese Komponenten, wie zum Beispiel eine Faltschachtel. Darin werden die Komponenten jeweils getrennt in verschiedenen Containern bereitgestellt. Unter Container wird im Rahmen der vorliegenden Erfindung eine Umhüllung verstanden, die in Form einer gegebenenfalls wieder-verschließbaren Flasche, einer Tube, einer Dose, eines Tütchens, eines Sachets oder einer ähnlichen Umhüllung vorliegt. Dem Umhüllungsmaterial sind erfindungsgemäß keine Grenzen gesetzt. Bevorzugt handelt es sich jedoch dabei um Umhüllungen aus Glas oder Kunststoff.

Darüber hinaus kann die Verpackungseinheit Applikationshilfen, wie Kämme, Bürsten oder Pinsel, persönliche Schutzkleidung, insbesondere Ein-Weg-Handschuhe, sowie eine Gebrauchsanleitung umfassen.

Unter einem kosmetisch akzeptablen Trägermedium ist im Sinne der vorliegenden Erfindung eine flüssige oder feste Zubereitung zu verstehen, welche geeignet ist, die Wirkstoffe unter den Lagerbedingungen zu stabilisieren. Der kosmetische Träger ist dabei bevorzugt wässrig, alkoholisch oder wässrig-alkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Ein wässriger Träger enthält im Sinne der Erfindung mindestens 30 Gew.-%, insbesondere mindestens 50 Gew.-% Wasser, bezogen auf das Gesamtgewicht der Komponente. Unter alkoholischen Trägern sind im Sinne der vorliegenden Erfindung Zusammensetzungen, enthaltend mindestens 50 Gew.-% eines $C_1$-$C_4$-Alkohols, bezogen auf das Gesamtgewicht der Komponente, insbesondere Ethanol bzw. Isopropanol, zu verstehen. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Komponente, insbesondere Ethanol bzw. Isopropanol, zu verstehen. Die erfindungsgemäßen Komponenten können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Die erste Komponente (I) des erfindungsgemäß verwendeten Kits enthält als wesentlichen Inhaltsstoff mindestens eine organische Thiol-Verbindung. Es ist bevorzugt, wenn diese Thiol-Verbindung noch mindestens eine weitere funktionelle Gruppe, ausgewählt aus einer Aminogruppe, einer Hydroxygruppe oder einer Carbonsäuregruppe besitzt. Besitzt die Thiol-Verbindung zusätzlich eine Carbonsäuregruppe, ist dies ganz besonders bevorzugt.

Die organische Thiol-Verbindung kann dabei als ungeladene Verbindung eingesetzt werden. Sofern die Thiol-Verbindung protonierbare oder deprotonierbare Struktureinheiten enthält, ist ebenso möglich, diese als physiologisch verträgliches Salz oder, sofern möglich, als zwitterionisches inneres Salz einzusetzen.

Insbesondere Carbonsäuregruppen-haltige Thiol-Verbindungen liegen bevorzugt in Form der entsprechenden Carboxylat-Verbindung vor.

Als Carboxylatgruppe wird eine deprotonierte Carbonsäuregruppe bezeichnet, welche nach Abspaltung des aciden Protons negativ geladen ist. Zur Neutralisierung der negativen Ladung und zum Gesamtladungsausgleich muss ein positiv geladenes Gegenion vorliegen. Begünstigt ist es, wenn es sich bei dem positiven Gegenion um ein Alkalimetallkation, insbesondere Natrium ($Na^+$) oder Kalium ($K^+$), ein Ammoniumion ($NH_4^+$) oder ein positiv geladenes organisches Ammoniumion (z.B. $HO\text{-}CH_2\text{-}CH_2\text{-}NH_3^+$) handelt.

Beispielhafte und besonders geeignete Thiol-Verbindungen der Komponente (I) sind Cystein, Cystein Hydrochlorid, Thioglykolsäure, Ammoniumthioglykolat, Ethanolamin Thioglykolat und Thiomilchsäure.

Eine Ausführungsform des ersten Erfindungsgegenstands ist daher eine Verfahren, welches dadurch gekennzeichnet ist, dass die organische Thiol-Verbindung der Komponente (I) ausgewählt ist aus der Gruppe, die gebildet wird aus Cystein, Thioglykolsäure, Thiomilchsäure und/oder deren physiologisch verträglichen Salzen.

Physiologisch verträgliche Salze sind dabei die bereits genannten Deprotonierungsprodukte von Carbonsäuren, aber auch kationische Verbindungen, bei denen insbesondere Amin-Gruppen in der Thiol-Verbindung mittels geeigneter Säuren, insbesondere Mineralsäuren wie Salzsäure, Bromwasserstoff oder Schwefelsäure, organischen Säuren, wie Citronensäure, Weinsäure oder Essigsäure, oder organischen Sulfonsäuren, wie p-Toluolsulfonsäure, Benzolsulfonsäure oder Methansulfonsäure, in die entsprechenden Protonierungsprodukte überführt werden.

Besonders bevorzugt als organische Thiol-Verbindung sind Cystein sowie Cystein Hydrochlorid.

Bevorzugt enthält die Komponente (I) eine oder mehrere organische Thiol-Verbindungen in einem Gewichtsanteil von 0,1 bis 20 Gew.-%, bevorzugt 0,5 bis 15 Gew.-%, weiter bevorzugt von 1,0 bis 10 Gew.-% und besonders bevorzugt von 2,0 bis 8,0 Gew.-%, jeweils bezogen auf das Gesamtgewicht der Komponente (I).

Die Komponente (I), enthaltend die organische Thiol-Verbindung, kann in flüssiger oder pastöser Form, jeweils bevorzugt auf wässriger Basis, vorliegen. Der pH-Wert dieser Formulierung kann auf einen Wert von pH 2 bis pH 11 eingestellt sein. Bei den pH-Werten im Sinne der vorliegenden Erfindung handelt es sich um pH-Werte, die bei Raumtemperatur (ca. 22 °C) gemessen wurden. Zur Einstellung des pH-Werts sind dem Fachmann gängige Acidifizierungs- und Alkalisierungsmittel geläufig. Die zur Einstellung des pH-Wertes verwendbaren Alkalisierungsmittel werden typischerweise gewählt aus Ammoniak, anorganischen Salzen, insbesondere der Alkali- und Erdalkalimetalle, organischen Alkalisierungsmitteln, insbesondere Alkanolaminen, Aminen und basischen Aminosäuren. Erfindungsgemäß bevorzugte Acidifizierungsmittel sind Genuss-Säuren, wie Milchsäure, Zitronensäure, Essigsäure, Äpfelsäure oder Weinsäure, verdünnte Mineralsäuren und deren in Wasser sauer reagierenden Salze sowie organische Phosphon- oder Sulfonsäuren. Zur Stabilisierung des pH-Werts können weiterhin entsprechende pH-Puffersysteme zugesetzt werden.

Im Rahmen einer Ausführungsform ist es besonders bevorzugt, wenn die Komponente (I), enthaltend mindestens eine organische Thiol-Verbindung, einen neutralen bis sauren pH-Wert von pH 2 bis 7, insbesondere von pH 2 bis 6 besitzt. Im Rahmen einer weiteren bevorzugten Ausführungsform ist es besonders vorteilhaft, die Komponente (I) auf einen mild alkalischen pH-Wert von pH 7,5 bis 9,8 einzustellen.

Eine Ausführungsform des ersten Erfindungsgegenstands ist daher dadurch gekennzeichnet, dass die Komponente (I) einen pH-Wert von pH 2 bis pH 6 oder einen pH-Wert von pH 7,5 bis pH 9,8 besitzt.

Die Komponente (II) des erfindungsgemäß verwendeten Kits ist dadurch gekennzeichnet, dass sie als wesentlichen Bestandteil mindestens ein Persulfatsalz enthält. Vorzugsweise ist dieses Persulfatsalz ausgewählt aus Ammoniumperoxodisulfat, Alkalimetallperoxodisulfaten, Ammoniumperoxomonosulfat und Alkalimetallhydrogenperoxomonosulfaten. Besonders bevorzugt sind Ammoniumperoxodisulfat, Kaliumperoxodisulfat, Natriumperoxodisulfat und Kaliumhydrogenperoxomonosulfat.

Weiterhin hat es sich bei den Arbeiten zur vorliegenden Erfindung als besonders bevorzugt erwiesen, wenn die erfindungsgemäß verwendeten Mittel mindestens zwei verschiedene Peroxodisulfate enthalten. Bevorzugte Peroxodisulfatsalze sind dabei Kombinationen aus Ammoniumperoxodisulfat und Kaliumperoxodisulfat und/oder Natriumperoxodisulfat.

Eine Ausführungsform des ersten Erfindungsgegenstands ist daher eine Verfahren, das dadurch gekennzeichnet ist, dass die Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält.

Aus Gründen der Lagerstabilität liegt die Komponente (II) vorteilhafterweise in Pulverform vor und kann zusätzlich Stabilisatoren, Alkalitätsgeber, Quellmittel, pflanzliche Gummen, fein gemahlene mineralische Produkte wie Magnesiumcarbonat, Magnesiumoxid oder Bariumoxid und/oder Löslichkeitsverbesserer enthalten. Ebenso können zusätzliche Alkalisierungsmittel wie Alkalimetallcarbonate und insbesondere Alkalimetallsilikate und -metasilikate in die Komponente (II) eingearbeitet werden. Zur Entstaubung der Pulver können der Komponente (II) entsprechende Entstaubungsmittel, insbesondere Öle, wie Paraffin, Siliconöle oder Esteröle, zugesetzt werden.

Bevorzugt enthält die Komponente (II) eine oder mehrere Persulfatsalze in einem Gewichtsanteil von 20 bis 100 Gew.-%, bevorzugt 40 bis 100 Gew.-% und besonders bevorzugt von 50 bis 100 Gew.-%, jeweils bezogen auf das Gesamtgewicht der Komponente (II).

Das erfindungsgemäß verwendete Kit umfasst als dritte, getrennt konfektioniert vorliegende Komponente (III) eine Formulierung, die Wasserstoffperoxid enthält. Erfindungsgemäß bevorzugt wird Wasserstoffperoxid in dem erfindungsgemäßen Kit als wässrige Wasserstoffperoxid-Lösung eingesetzt.

Die Konzentration einer Wasserstoffperoxid-Lösung wird einerseits von den gesetzlichen Vorgaben und andererseits von dem gewünschten Effekt bestimmt; vorzugsweise werden 6 bis 12 Gew.-%-ige Lösungen in Wasser verwendet. Erfindungsgemäß bevorzugte Komponenten (III) sind dadurch gekennzeichnet, dass sie, bezogen auf ihr Gesamtgewicht, 0,01 bis 12 Gew.-%, vorzugsweise 0,1 bis 10 Gew.-%, besonders bevorzugt 1 bis 6 Gew.-% Wasserstoffperoxid, (berechnet als 100%iges $H_2O_2$) enthalten.

Die Komponente (III) besitzt zur Stabilisierung des Wasserstoffperoxids bevorzugt einen schwach sauren pH-Wert, insbesondere einen pH-Wert von pH 2,5 bis pH 6,5, insbesondere von pH 3 bis pH 5.

Um die Aktivität des Wasserstoffperoxids zu verbessern und die Blondierwirkung auf der keratinischen Faser zu verstärken, ist es jedoch vorteilhaft, wenn die Anwendungszubereitung mit Wasserstoffperoxid einen alkalischen pH-Wert besitzt. Daher ist es erfindungsgemäß bevorzugt, wenn die Mehrkomponentenverpackungseinheit eine weitere Komponente (IV), enthaltend mindestens ein Alkalisierungsmittel, umfasst.

Diese Komponente (IV) wird unmittelbar vor der Anwendung auf der keratinischen Faser mit der Komponente (III), enthaltend Wasserstoffperoxid, zur anwendungsbereiten Zubereitung vermischt. Das Mischverhältnis zwischen Komponente (III) und (IV) beträgt üblicherweise von 10 zu 1 bis 1 zu 10 Gewichtsteilen, bevorzugt von 3 zu 1 bis 1 zu 3 Gewichtsteilen und insbesondere 1 zu 1 Gewichtsteile.

Eine weitere, bevorzugte Ausführungsform des ersten Erfindungsgegenstands ist daher eine Verfahren, das dadurch gekennzeichnet ist, dass die Mehrkomponentenverpackungseinheit zusätzlich

iv) eine vierte Komponente (IV), enthaltend in einem kosmetisch akzeptablen Trägermedium mindestens ein Alkalisierungsmittel, ausgewählt aus der Gruppe, die gebildet wird aus Ammoniak, Monoethanolamin, 2-Amino-2-methylpropanol, Triethanolamin, Natrium-hydroxid und Kaliumhydroxid, enthält.

Der Anteil an Alkalisierungsmittel in der Komponente (IV) ist bevorzugt ausreichend, um die anwendungsbereite Zubereitung aus den Komponenten (III) und (IV) auf einen pH-Wert von pH 8 bis 11, bevorzugt 8,5 bis 10,5 einzustellen.

Zur verbesserten Vermischbarkeit der Komponenten (III) und (IV) sowie besseren Anwendungseigenschaften der resultierenden Zubereitung enthält die Komponente (IV) bevorzugt eine grenzflächenaktive Verbindung, insbesondere ein Tensid. Dabei sind anionische, nichtionische, zwitterionische und amphotere Tenside besonders geeignet.

Eine besonders bevorzugte Ausführungsform des ersten Erfindungsgegenstands ist daher eine Verfahren, welches dadurch gekennzeichnet ist, dass die Komponente (IV) zusätzlich mindestens ein anionisches, nichtionisches, zwitterionisches und/oder amphoteres Tensid enthält.

Als anionische Tenside eignen sich in erfindungsgemäß verwendeten Zubereitungen alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind lineare und verzweigte Fettsäuren mit 8 bis 30 C-Atomen (Seifen), Alkylethercarbonsäuren, Acylsarcoside, Acyltauride, Acylisethionate, Sulfobernsteinsäuremono-, -dialkylester und Sulfobernsteinsäuremono-alkylpolyoxyethylester, lineare Alkansulfonate, lineare $\alpha$-Olefinsulfonate, Alkylsulfate und Alkylethersulfate sowie Alkyl- und/oder Alkenylphosphate. Bevorzugte anionische Tenside sind Alkylsulfate, Alkylethersulfate und Alkylethercarbonsäuren mit jeweils 10 bis 18 C-Atomen in der Alkylgruppe und bis zu 12 Glykolethergruppen im Molekül. Beispiele solcher Tenside sind die Verbindungen mit den INCI-Bezeichnungen Sodium Laureth Sulfate, Sodium Lauryl Sulfate, Sodium Myreth Sulfate oder Sodium Laureth Carboxylate.

Als zwitterionische Tenside werden solche oberflächenaktiven Verbindungen bezeichnet, die im Molekül mindestens eine quartäre Ammoniumgruppe und mindestens eine Carboxylat-, Sulfonat-oder Sulfat-Gruppe tragen. Besonders geeignete zwitterionische Tenside sind die so genannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine -COOH- oder-$SO_3H$-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und das $C_{12}$-$C_{18}$-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise Anlagerungsprodukte von 4 bis 50 Mol Ethylenoxid und/oder 0 bis 5 Mol Propylenoxid an lineare und verzweigte Fettalkohole, an Fettsäuren und an Alkylphenole, jeweils mit 8 bis 20 C-Atomen in der Alkylgruppe, ethoxylierte Mono-, Di- und Triglyceride, wie beispielsweise Glycerinmonolaurat + 20 Ethylenoxid, und Glycerinmonostearat + 20 Ethylenoxid, Sorbitanfettsäureester und Anlagerungeprodukte von Ethylenoxid an Sorbitanfettsäureester wie beispielsweise die Polysorbate (TWEEN 20, TWEEN 21, TWEEN 60, TWEEN 61, TWEEN 81), Anlagerungsprodukte von Ethylenoxid an Fettsäurealkanolamide und Fettamine, sowie Alkylpolyglycoside. Als nichtionische Tenside eignen sich insbesondere $C_8$-$C_{22}$-Alkylmono-und -oligoglycoside und deren ethoxylierte Analoga sowie Alkylenoxid-Anlagerungsprodukte an gesättigte oder ungesättigte lineare Fettalkohole mit jeweils 2 bis 30 Mol Ethylenoxid pro Mol Fettalkohol.

Die anionischen, nichtionischen, zwitterionischen oder amphoteren Tenside werden in Mengen von 0,1 bis 50 Gew.%, bevorzugt 1 bis 30 Gew.% und ganz besonders bevorzugt von 1 bis 25 Gew.%, bezogen auf die Gesamtmenge der Komponente (IV), eingesetzt.

Eine bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

> i) die erste Komponente (I) in einem kosmetisch akzeptablen Trägermedium mindestens Cystein und/oder Cystein Hydrochlorid enthält,
> ii) die zweite Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält,
> iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und
> iv) die Komponente (IV) in einem kosmetisch akzeptablen Trägermedium mindestens ein Alkalisierungsmittel, ausgewählt aus der Gruppe, die gebildet wird aus Ammoniak, Monoethanolamin, 2-Amino-2-methylpropanol, Triethanolamin, Natriumhydroxid und Kaliumhydroxid, enthält.

Eine bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

> i) die erste Komponente (I) in einem kosmetisch akzeptablen Trägermedium mindestens Cystein und/oder Cystein Hydrochlorid enthält,
> ii) die zweite Komponente (II) mindestens Kaliumperoxodisulfat enthält,
> iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und
> iv) die Komponente (IV) in einem kosmetisch akzeptablen Trägermedium mindestens ein Alkalisierungsmittel, ausgewählt aus der Gruppe, die gebildet wird aus Ammoniak, Monoethanolamin, 2-Amino-2-methylpropanol, Triethanolamin, Natriumhydroxid und Kaliumhydroxid, enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

> i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,
> ii) die zweite Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält,
> iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und
> iv) die Komponente (IV) in einem kosmetisch akzeptablen Trägermedium als Alkalisierungsmittel mindestens Ammoniak und/oder Monoethanolamin enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

> i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,
> ii) die zweite Komponente (II) mindestens Kaliumperoxodisulfat enthält,
> iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und
> iv) die Komponente (IV) in einem kosmetisch akzeptablen Trägermedium als Alkalisierungsmittel mindestens Ammoniak und/oder Monoethanolamin enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,

ii) die zweite Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält,

iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und

iv) die Komponente (IV) in einem kosmetisch akzeptablen Trägermedium als Alkalisierungsmittel mindestens Ammoniak und/oder Monoethanolamin sowie zusätzlich mindestens ein anionisches und/oder nichtionisches Tensid enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,

ii) die zweite Komponente (II) mindestens Kaliumperoxodisulfat enthält,

iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und

iv) die Komponente (IV) in einem kosmetisch akzeptablen Trägermedium als Alkalisierungsmittel mindestens Ammoniak und/oder Monoethanolamin sowie zusätzlich mindestens ein anionisches und/oder nichtionisches Tensid enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,

ii) die zweite Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält,

iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und

iv) die Komponente (IV) in einem kosmetisch akzeptablen Trägermedium als Alkalisierungsmittel mindestens Ammoniak und/oder Monoethanolamin sowie zusätzlich mindestens einen Gewichtsanteil von 1,0 bis 15 Gew.-%, bezogen auf das Gesamtgewicht der Komponente (IV), von anionischen und/oder nichtionischen Tensiden enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,

ii) die zweite Komponente (II) mindestens Kaliumperoxodisulfat enthält,

iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und

iv) die Komponente (IV) in einem kosmetisch akzeptablen Trägermedium als Alkalisierungsmittel mindestens Ammoniak und/oder Monoethanolamin sowie zusätzlich mindestens einen Gewichtsanteil von 1,0 bis 15 Gew.-%, bezogen auf das Gesamtgewicht der Komponente (IV), von anionischen und/oder nichtionischen Tensiden enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,
ii) die zweite Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält,
iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und
iv) die Komponente (IV) in einem kosmetisch akzeptablen Trägermedium als Alkalisierungsmittel mindestens Ammoniak und/oder Monoethanolamin sowie als zusätzliches anionisches Tensid mindestens ein Alkylethersulfat und/oder eine Alkylethercarbonsäure enthält.

Erne weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,
ii) die zweite Komponente (II) mindestens Kaliumperoxodisulfat enthält,
iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und
iv) die Komponente (IV) in einem kosmetisch akzeptablen Trägermedium als Alkalisierungsmittel mindestens Ammoniak und/oder Monoethanolamin sowie als zusätzliches anionisches Tensid mindestens ein Alkylethersulfat und/oder eine Alkylethercarbonsäure enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,
ii) die zweite Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält,
iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und
iv) die Komponente (IV) in einem kosmetisch akzeptablen Trägermedium als Alkalisierungsmittel mindestens Ammoniak und/oder Monoethanolamin sowie als zusätzliches nichtionisches Tensid mindestens ein $C_8$-$C_{22}$-Alkylmono- und -oligoglycosid und/oder ein Ethylenoxid-Anlagerungsprodukt an gesättigte oder ungesättigte lineare Fettalkohole mit jeweils 2 bis 30 Mol Ethylenoxid pro Mol Fettalkohol enthält.

Eine weitere bevorzugte Ausführungsform des ersten Erfindungsgegenstands ist ein Verfahren zur Aufhellung keratinischer Fasern unter Einsatz einer Mehrkomponentenverpackungseinheit, enthaltend vier getrennt voneinander konfektionierte Komponenten, dadurch gekennzeichnet, dass

i) die erste Komponente (I) in einem kosmetisch akzeptablen Trägermedium mindestens Cystein und/oder Cystein Hydrochlorid in einem Gewichtsanteil von 2 bis 8 Gew.-%, bezogen auf das Gesamtgewicht der Komponente (I), enthält,

ii) die zweite Komponente (II) mindestens Kaliumperoxodisulfat enthält,

iii) die dritte Komponente (III) in einem kosmetisch akzeptablen Trägermedium Wasserstoffperoxid in einem Anteil von 0,01 bis 12 Gew.-%, bezogen auf das Gesamtgewicht der Komponente (III) enthält, und

iv) die Komponente (IV) in einem kosmetisch akzeptablen Trägermedium als Alkalisierungsmittel mindestens Ammoniak und/oder Monoethanolamin sowie als zusätzliches nichtionisches Tensid mindestens ein $C_8$-$C_{22}$-Alkylmono- und -oligoglycosid und/oder ein Ethylenoxid-Anlagerungsprodukt an gesättigte oder ungesättigte lineare Fettalkohole mit jeweils 2 bis 30 Mol Ethylenoxid pro Mol Fettalkohol enthält.

Die Komponenten (I), (II) und (III) sowie gegebenenfalls (IV) der Mehrkomponentenverpackungseinheit des ersten Erfindungsgegenstands können weitere kosmetische Wirk- und Hilfsstoffe enthalten.

Die anwendungsbereiten Zubereitungen besitzen bevorzugt eine Viskosität, die es ermöglicht, dass das Mittel sich einerseits gut auftragen lässt und andererseits über ein solches Fließverhalten verfügt, dass es für das Mittel eine ausreichend lange Einwirkzeit am Wirkort garantiert.

So hat es sich als vorteilhaft erwiesen, wenn die Komponenten mindestens ein Verdickungsmittel zur Einstellung der Viskosität der Zubereitungen enthalten. Bezüglich dieser Verdickungsmittel bestehen keine prinzipiellen Einschränkungen. Es können sowohl organische als auch rein anorganische Verdickungsmittel zum Einsatz kommen.

Geeignete zusätzliche Verdickungsmittel sind

- nichtionische, synthetische Polymere, wie beispielsweise Vinylpyrrolidinon/Vinylacrylat-Copolymere, Polyvinylpyrrolidinon, Polyethylenglykole und Polysiloxane;
- anionische, synthetische Polymere, wie beispielsweise Polyacrylsäuren oder vernetzte Polyacrylsäuren;
- kationische, synthetische Polymere, wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Ferner können die erfindungsgemäß verwendeten Mittel weitere Wirk-, Hilfs- und Zusatzstoffe enthalten, beispielsweise kationische Tenside, wie Cetyltrimethylammoniumchlorid, Quaternium-27 oder N,N-Bis(2-Palmitoyloxyethyl)dimethylammoniumchlorid; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide, insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffel- und Weizenproteinhydrolysate, sowie in Form ihrer Fettsäure-Kondensationsprodukte oder gegebenenfalls anionisch oder kationisch modifizierten Derivate; pflanzliche Öle, wie Macadamianussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, $B_3$, $B_5$, $B_6$, C, E, F und H; Pflanzenextrakte; natürliche oder synthetische Farbstoffe und Pigmente; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Komplexbildner, wie EDTA, EDDS, HEDP oder deren Salze; weitere pH-Stellmittel, insbesondere Acidifizierungsmittel wie Genuss-Säuren, beispielsweise Zitronensäure, Essigsäure, Äpfelsäure oder Weinsäure, sowie verdünnte Mineralsäuren, und Alkalisierungsmittel wie anorganischen Salzen, insbesondere der Alkali- und Erdalkalimetalle, organischen Alkalisierungsmitteln, insbesondere Aminen, basische Aminosäuren und Alkanolaminen, und Ammoniak; sowie Treibmittel wie Pröpan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Die zusätzlichen Wirk- und Hilfsstoffe werden in den erfindungsgemäß verwendeten Mitteln bevorzugt in Mengen von jeweils 0,0001 bis 25 Gew.-%, insbesondere von 0,0005 bis 15 Gew.-%, bezogen auf das Gesamtgewicht der Komponenten, eingesetzt.

Die Komponenten der Mehrkomponentenverpackungseinheit des ersten Erfindungsgegenstands werden zur Aufhellung und oder Blondierung von keratinischen Fasern, einzeln und nacheinander oder gemeinsam und gleichzeitig oder teilweise gemeinsam und gleichzeitig und teilweise nacheinander aufgetragen.

Bevorzugt beträgt die Einwirkzeit 5 bis 60 min, insbesondere 5 bis 50 min, besonders bevorzugt 10 bis 45 min. Während der Einwirkzeit der Mittel auf der Faser kann es vorteilhaft sein, den Aufhellvorgang durch Wärmezufuhr zu unterstützen. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Mittel ergeben bereits bei physiologisch verträglichen Temperaturen von unter 45°C gute Behandlungsergebnisse.

Nach Ende der Einwirkzeit wird das verbleibende Mittel mit einer wässrigen Zubereitung, insbesondere mit Wasser selbst oder einem tensidhaltigen Reinigungsmittel aus dem Haar gespült. Als Reinigungsmittel kann dabei insbesondere ein handelsübliches Shampoo dienen, wobei insbesondere dann auf das Reinigungsmittel verzichtet werden kann und der Ausspülvorgang mit Leitungswasser erfolgen kann, wenn die Anwendungszubereitung ein stark tensidhaltiges Trägermedium besitzt.

Eine besonders starke Verbesserung der Aufhellleistung erfolgt vor allem dann, wenn die Komponente (I) des Kits, enthaltend die organische Thiol-Verbindung, zunächst in einem Vorbehandlungsschritt auf die noch trockenen oder bereits mit Wasser angefeuchteten Keratinfasern aufgebracht und dort für 5 bis 45 Minuten belassen wird.

Anschließend wird die Komponente (I) vor Aufbringen der weiteren Komponenten (II) und (III) sowie gegebenenfalls (IV) aus dem Haar ausgespült.

In einem nächsten Schritt wird die Komponente (III), enthaltend Wasserstoffperoxid, mit der Komponente (II), enthaltend mindestens ein Persulfatsalz, sowie gegebenenfalls der Komponente (IV) miteinander vermischt, diese Mischung auf die Haarfaser aufgebracht und dort für 10 bis 45 Minuten, gegebenenfalls unter Einsatz einer äußeren Wärmequelle, belassen.

Die Anwendungsmischung aus den Komponenten (II) und (III) sowie gegebenenfalls (IV) besitzt bevorzugt einen alkalischen pH-Wert von 8 bis 11,5. Dieser pH-Wert kann beispielsweise durch weitere Zusätze von Metasilikate, Phosphate oder auch Natriumcarbonat eingestellt werden.

Zur Erzielung eines verbesserten Blondierergebnisses ohne gleichzeitige Haarschädigung wird die Anwendungsmischung aus den Komponenten (II) und (III) sowie gegebenenfalls (IV) bevorzugt aus Anteilen der jeweiligen Komponenten so zusammengemischt, dass der Anteil an Persulfat-Salzen in der Mischung zwischen 1 und 30 Gew.-%, bevorzugt 3 und 25 Gew.-% und besonders bevorzugt 5 bis 20 Gew.-%, jeweils bezogen auf das Gesamtgewicht der Mischung der Komponenten (II) und (III) sowie gegebenenfalls (IV), beträgt.

Nach Ende des Blondiervorgangs werden alle auf den Keratinfasern befindlichen Komponenten mit Wasser oder einem tensidhaltigen Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensidhaltigen Träger besitzt.

Zwischen der Anwendung der Komponente (I) und dem Aufbringen der Mischung aus den Komponenten (II) und (III) sowie gegebenenfalls (IV) kann ein Zeitraum von wenigen Minuten bis zu einigen Stunden liegen, bevorzugt erfolgen die Verfahrensschritte aber direkt nach Beendigung des Ausspülvorgangs der Komponente (I).

Bevorzugt ist dabei jeweils ein Verfahren, bei dem die Komponente (I) für 10 bis 30 min auf den Keratinfasern belassen wird. Bevorzugt ist dabei weiterhin ein Verfahren, bei dem Keratinfasern mit der aus den Komponenten (II) und (III) sowie gegebenenfalls (IV) hergestellten Mischung für 20 bis 40 min behandelt werden.

BEISPIELE

1. Formulierungen

(soweit nicht anders angegeben entsprechen die Mengenangaben Gewichtsanteilen)

1.1 Herstellung der Komponente (I)

Es wurden die folgenden Lösungen hergestellt:

| Komponente | Vorbehandlungslösung (I)A | Vorbehandlungslösung (I)B |
|---|---|---|
| Cystein | 2,5 Gew.-% | 5,0 Gew.-% |

(fortgesetzt)

| Komponente | Vorbehandlungslösung (I)A | Vorbehandlungslösung (I)B |
|---|---|---|
| Ammoniak | ad pH 9,5 | ad pH 9,5 |
| Wasser | ad 100 | ad 100 |

Als Komponente (I) wurden weiterhin die nachfolgenden Formulierungen hergestellt:

| | Komponente (I)C [Gew.-%] | Komponente (I)D [Gew.-%] |
|---|---|---|
| Stenol® 16/18 | 4,0 | 4,0 |
| Eumulgin® B1 | 1,0 | 1,0 |
| Dehyquart® A-CA | 2,0 | 2,0 |
| Ammoniumsulfat 1,0 g (in 30,0 g Wasser) | 1,0 | 1,0 |
| Ammoniumthioglykolat (59%ige wässrige Lösung) | -- | 10,0 |
| Cystein | 5,0 | -- |
| Ammoniak | ad pH 9,5 | ad pH 9,5 |
| Wasser | ad 100 | ad 100 |

| | |
|---|---|
| Dehyquart® A-CA | Trimethylhexadecylammoniumchlorid (ca. 24-26 % Aktivsubstanz; INCI-Bezeichnung: Aqua, Cetrimonium Chloride; Cognis) |
| Eumulgin® B 1 | Cetylstearylalkohol mit ca. 12-EO-Einheiten (INCI-Bezeichnung: Ceteareth-12; Cognis) |
| Stenol® 1618 | $C_{16-18}$-Fettalkohol (INCI-Bezeichnung: Cetearyl Alcohol; Cognis) |

Das Stenol® 16/18 wurde zusammen mit Eumulgin® B1 und Dehyquart® A-CA aufgeschmolzen, danach wurde die Schmelze mit heißem Wasser emulgiert. Dann wurden Cystein bzw. Ammoniumthioglykolat-Lösung sowie die wässrige Ammoniumsulfatlösung hinzugegeben und mit Wasser auf ca. 90 g aufgefüllt. Mit Ammoniak wurde ein pH-Wert von 9,5 eingestellt, anschließend wurde mit Wasser auf 100 g aufgefüllt.

1.2. Herstellung der Komponente (II)

Als Komponente (II) wurde Kaliumperoxodisulfat oder ein Gemisch aus Ammoniumperoxodisulfat, Natriumperoxodisulfat und Kaliumperoxodisulfat (je ein Gewichtsteil) verwendet.

1.3 Herstellung der Komponente (III) (Entwicklerdispersion)

| Rohstoff | Gew.-% |
|---|---|
| Ammoniak, 25 % | 0,62 |
| Dipicolinsäure | 0,10 |
| Dinatriumpyrophosphat | 0,03 |
| Turpinal SL | 1,50 |
| Texapon NSO | 2,00 |
| Dow Corning DB 110 A (nichtionische Silikonemulsion) | 0,07 |
| Aculyn 33A (Acrylpolymer) | 12,00 |

(fortgesetzt)

| Rohstoff | Gew.-% |
|---|---|
| Wasserstoffperoxid 50 % | 22,40 |
| Wasser | ad 100 |

Aus den aufgelisteten Bestandteilen wurde eine Blondiercreme (Komponente (IV)) wie folgt hergestellt:

| Rohstoff | Gew.-% |
|---|---|
| Hydrenol D | 12,00 |
| Lorol. techn. | 2,40 |
| Texapon NSO | 26,50 |
| Stabylen 30 | 0,10 |
| Cetiol OE | 2,40 |
| Turpinal SL | 0,20 |
| Natriumsilikat 40/42 | 0,50 |
| Ammoniumsulfat | 1,00 |
| Ammoniak, 25% | 7,60 |
| Wasser | ad 100 |

| | |
|---|---|
| Aculyn® 33A | ca. 28% Festkörper in Wasser; INCI-Bezeichnung: Acrylates Copolymer (Rohm&Haas) |
| Hydrenol® D | INCI-Bezeichnung: Cetearyl alcohol (Cognis) |
| Lorol® tech. | INCI-Bezeichnung: Coconut alcohol (Cognis) |
| Texapon® NSO | ca. 27,5% Aktivsubstanz; INCI-Bezeichnung: Sodium Laureth Sulfate (Cognis) |
| Stabylen® 30 | INCI-Bezeichnung: Acrylates/Vinylisodecanoate Crosspolymer (3V Sigma) |
| Cetiol OE | INCI-Bezeichnung: Dicaprylether (Cognis) |
| Turpinal® SL | ca. 58-61% Aktivsubstanzgehalt; INCI-Bezeichnung: Etidronic Acid, Aqua (Solutia) |

Hydrenol D und Lorol wurden zusammen bei 80 °C aufgeschmolzen, dann wurden Texapon NSO, Stabylen 30, Cetiol OE und Turpinal SL der Reihe nach unter Rühren eingearbeitet. Natriumsilikat 40/42 und Ammoniumsulfat wurden jeweils in einer kleinen Menge Wasser vorgelöst und ebenfalls unter Rühren hinzugefügt. Bei einer Temperatur von ca. 40 °C wurde schließlich der Ammoniak zugegeben. Unter Rühren wurde mit Wasser auf 100 % aufgefüllt und die Formulierung kalt gerührt.

Die Blondiercreme (IV) wurde im Gewichts-Verhältnis 1:1 mit der Entwicklerdispersion (III) zu einer frisch hergestellten Zubereitung aus (III) und (IV) vermischt.

2. Anwendung

2.1. Anwendungsbeispiel 1

Für den Vorbehandlungsschritt mit Komponente (I) wurden Strähnen dunkelblonden, hellbraunen und dunkelbraunen Haares (Codes: Kerling 7/0, Fischbach & Miller 6923 und Kerling 2/0) von ca. 0,7 g Gewicht mit jeweils ca. 10 ml der Komponente (I)B (5 Gew.-% Cystein) behandelt. Nach einer Einwirkzeit von 20 Minuten bei 32 °C wurden die Strähnen mit einem handelsüblichen Shampoo für ca. 1 Minute ausgewaschen und mit einem Handtuch getrocknet. Die Strähnen wurden in handtuchtrockenem Zustand sofort weiterbehandelt.

100 g der frisch hergestellten Zubereitung aus (III) und (IV), enthaltend Wasserstoffperoxid, wurden mit 8,33 g der Komponente (II) bestehend aus Kaliumperoxodisulfat vermischt. Für den Blondierprozess wurde auf die vorbehandelten Haarsträhnen die 4-fache Menge dieser Anwendungsmischung appliziert. Nachdem die Strähnen für 30 min bei 32 °C blondiert wurden, wurden sie mit einem handelsüblichen Shampoo gewaschen und mit einem Föhn getrocknet.

Jede Haarsträhne wurde vor und nach dem Bleichvorgang farbmetrisch mit einem Farbmessgerät der Firma Datacolor, Typ Spectraflash 450 vermessen. Als Maß für die Aufhellleistung der jeweiligen Rezeptur wurde der $\Delta$L-Wert gemäß folgender Formel herangezogen:

$$\Delta L = L_{nachher} - L_{vorher}$$

$L_{nachher} =$ Helligkeit der Strähne nach dem Bleichen
$L_{vorher} =$ Helligkeit der Strähne vor dem Bleichen

Für jede Rezeptur und jeden Haartyp erfolgte eine Doppelbestimmung, aus den Einzelwerten wurde jeweils der Mittelwert gebildet. Der $\Delta$L-Wert kann als direktes Maß für die Bleichwirkung der jeweiligen Rezeptur herangezogen werden.

Als nicht erfindungsgemäßer Vergleich wurden entsprechende Haarsträhnen dem gleichen Blondierprozess ohne den erfindungsgemäßen Vorbehandlungsschritt mit Komponente (I) unterzogen.

| Haartyp | $\Delta$L (ohne Vorbehandlung) | $\Delta$L (erfindungsgemäß, Vorbehandlung mit (I)B) |
|---|---|---|
| dunkelblond | 18,4 | 20,6 |
| hellbraun | 21,4 | 24,8 |
| dunkelbraun | 8,6 | 12,9 |

2.2. Anwendungsbeispiel 2

Die Behandlung der Haare erfolgte wie in Beispiel 1, allerdings wurden als Komponente (II) 20 g einer Mischung aus Ammoniumperoxodisulfat, Natriumperoxodisulfat und Kaliumperoxodisulfat (je ein Gewichtsteil) verwendet.

| Haartyp | $\Delta$L (ohne Vorbehandlung) | $\Delta$L (erfindungsgemäß, Vorbehandlung mit (I)B) |
|---|---|---|
| dunkelblond | 23,6 | 27,1 |
| hellbraun | 26,4 | 28,5 |
| dunkelbraun | 15,2 | 19,9 |

2.3. Anwendungsbeispiel 3

Für den Vorbehandlungsschritt mit Komponente (I) wurden Strähnen dunkelblonden, hellbraunen und dunkelbraunen Haares (Codes: Kerling 7/0, Fischbach & Miller 6923 und Kerling 2/0) von ca. 0,7 g Gewicht mit jeweils ca. 10 ml der Komponente (I)A (2,5% Cystein) behandelt. Nach einer Einwirkzeit von 20 Minuten bei 32 °C wurde die überschüssige Flüssigkeit aus den Haarsträhnen gedrückt, und die Haare wurden, ohne sie auszuwaschen, in handtuchtrockenem Zustand weiterbehandelt.

100 g der frisch hergestellten Zubereitung aus (III) und (IV) wurden mit 8,33 g der Komponente (II) bestehend aus Kaliumperoxodisulfat vermischt. Für den Blondierprozess wurde auf die vorbehandelten Haarsträhnen die 4-fache Menge dieser Anwendungsmischung appliziert. Nachdem die Strähnen für 30 min bei 32 °C blondiert wurden, wurden sie mit einem handelsüblichen Shampoo gewaschen und mit einem Föhn getrocknet.

Als nicht erfindungsgemäßer Vergleich wurden entsprechende Haarsträhnen dem gleichen Blondierprozess ohne den erfindungsgemäßen Vorbehandlungsschritt mit Komponente (I) unterzogen.

| Haartyp | $\Delta$L (ohne Vorbehandlung) | $\Delta$L (erfindungsgemäß, Vorbehandlung mit (I)A) |
|---|---|---|
| dunkelblond | 18,5 | 20,5 |
| hellbraun | 19,7 | 20,6 |
| dunkelbraun | 8,6 | 11,3 |

**EP 2 608 848 B1**

2.4. Anwendungsbeispiel 4

Die Behandlung der Haare erfolgte wie in Beispiel 3, allerdings wurden als Komponente (II) 20 g einer Mischung aus Ammoniumperoxodisulfat, Natriumperoxodisulfat und Kaliumperoxodisulfat (je ein Gewichtsteil) verwendet.

| Haartyp | $\Delta$L (ohne Vorbehandlung) | $\Delta$L (erfindungsgemäß, Vorbehandlung mit (I)A) |
|---|---|---|
| dunkelblond | 24,6 | 28,1 |
| hellbraun | 25,5 | 28,6 |
| dunkelbraun | 15,5 | 18,9 |

2.5. Anwendungsbeispiel 5

Die Behandlung der Haare erfolgte wie in Beispiel 4, allerdings wurde anstelle von Komponente (I)A jeweils ca. 10 ml der Komponente (I)B (5% Cystein) verwendet.

| Haartyp | $\Delta$L (ohne Vorbehandlung) | $\Delta$L (erfindungsgemäß, Vorbehandlung mit (I)B) |
|---|---|---|
| dunkelblond | 18,5 | 23,1 |
| hellbraun | 19,7 | 24,0 |
| dunkelbraun | 8,6 | 14,4 |

2.6. Anwendungsbeispiel 6

Die Behandlung der Haare erfolgte wie in Beispiel 5, allerdings wurden als Komponente (II) 20 g einer Mischung aus Ammoniumperoxodisulfat, Natriumperoxodisulfat und Kaliumperoxodisulfat (je ein Gewichtsteil) verwendet.

| Haartyp | $\Delta$L (ohne Vorbehandlung) | $\Delta$L (erfindungsgemäß, Vorbehandlung mit (I)B) |
|---|---|---|
| dunkelblond | 24,6 | 28,7 |
| hellbraun | 25,5 | 30,1 |
| dunkelbraun | 15,5 | 20,9 |

2.7. Anwendungsbeispiel 7

Strähnen dunkelblonden, hellbraunen und dunkelbraunen Haares von ca. 0,7 g Gewicht wurden mit jeweils der vierfachen Menge der oben beschriebenen Komponente (I)C behandelt. Nach einer Einwirkzeit von 20 Minuten bei 32 °C wurde die überschüssige Formulierung abgestreift. Nach ca. 40 Minuten wurden die Strähnen ohne zwischenzeitlichen Auswaschvorgang weiterbehandelt.

100 g der frisch hergestellten Zubereitung aus (III) und (IV), enthaltend Wasserstoffperoxid, wurden mit 8,33 g der Komponente (II), bestehend aus Kaliumperoxodisulfat, vermischt. Für den Blondierprozess wurde auf die vorbehandelten Haarsträhnen die 4-fache Menge dieser Anwendungsmischung appliziert. Nachdem die Strähnen für 30 min bei 32 °C blondiert wurden, wurden sie mit einem handelsüblichen Shampoo gewaschen und mit einem Föhn getrocknet.

Als nicht erfindungsgemäßer Vergleich wurden entsprechende Haarsträhnen dem gleichen Blondierprozess ohne den erfindungsgemäßen Vorbehandlungsschritt mit Komponente (I) unterzogen.

| Haartyp | $\Delta$L (ohne Vorbehandlung) | $\Delta$L (erfindungsgemäß, Vorbehandlung mit (I)C) |
|---|---|---|
| dunkelblond | 16,4 | 23,6 |
| hellbraun | 19,7 | 24,2 |
| dunkelbraun | 7,5 | 13,3 |

2.8. Anwendungsbeispiel 8

Die Behandlung der Haare erfolgte wie in Beispiel 7, allerdings wurden als Komponente (II) 20 g einer Mischung aus Ammoniumperoxodisulfat, Natriumperoxodisulfat und Kaliumperoxodisulfat (je ein Gewichtsteil) verwendet.

| Haartyp | ΔL (ohne Vorbehandlung) | ΔL (erfindungsgemäß, Vorbehandlung mit (I)C) |
|---|---|---|
| dunkelblond | 24,2 | 29,3 |
| hellbraun | 26,6 | 34,7 |
| dunkelbraun | 14,6 | 21,6 |

2.9. Anwendungsbeispiel 9

Strähnen dunkelblonden, hellbraunen und dunkelbraunen Haares von ca. 0,7 g Gewicht wurden mit jeweils der vierfachen Menge der oben beschriebenen Komponente (I)D behandelt. Nach einer Einwirkzeit von 20 Minuten bei 32 °C wurden die Strähnen unter Zuhilfenahme eines handelsüblichen Shampoos ausgespült. Nach dem Auswaschvorgang wurden die Strähnen im handtuchtrockenen Zustand direkt weiterbehandelt.

100 g der frisch hergestellten Zubereitung aus (III) und (IV), enthaltend Wasserstoffperoxid, wurden mit 8,33 g der Komponente (II), bestehend aus Kaliumperoxodisulfat, vermischt. Für den Blondierprozess wurde auf die vorbehandelten Haarsträhnen die 4-fache Menge dieser Anwendungsmischung appliziert. Nachdem die Strähnen für 30 min bei 32 °C blondiert wurden, wurden sie mit einem handelsüblichen Shampoo gewaschen und mit einem Föhn getrocknet.

Als nicht erfindungsgemäßer Vergleich wurden entsprechende Haarsträhnen dem gleichen Blondierprozess ohne den erfindungsgemäßen Vorbehandlungsschritt mit Komponente (I) unterzogen.

| Haartyp | ΔL (ohne Vorbehandlung) | ΔL (erfindungsgemäß, Vorbehandlung mit (I)D) |
|---|---|---|
| dunkelblond | 17,3 | 21,0 |
| hellbraun | 19,1 | 25,3 |
| dunkelbraun | 8,2 | 13,9 |

2.10. Anwendungsbeispiel 10

Die Behandlung der Haare erfolgte wie in Beispiel 9, allerdings wurden als Komponente (II) 20 g einer Mischung aus Ammoniumperoxodisulfat, Natriumperoxodisulfat und Kaliumperoxodisulfat (je ein Gewichtsteil) verwendet.

| Haartyp | ΔL (ohne Vorbehandlung) | ΔL (erfindungsgemäß, Vorbehandlung mit (I)D) |
|---|---|---|
| dunkelblond | 25,2 | 27,7 |
| hellbraun | 26,0 | 30,9 |
| dunkelbraun | 15,6 | 22,5 |

**Patentansprüche**

1.   Verfahren zur Aufhellung von keratinischen Fasern, insbesondere menschlichen Haaren, welches **dadurch gekennzeichnet ist, dass** die Komponenten einer Mehrkomponentenverpackungseinheit (Kit-of-Parts) zur Aufhellung von keratinischen Fasern auf die keratinhaltigen Fasern aufgebracht, 5 bis 60 Minuten auf der Faser belassen und anschließend die Fasern mit Wasser gespült werden oder einem tensidhaltigen Reinigungsmittel ausgewaschen werden,
wobei die Mehrkomponentenverpackungseinheit mindestens die folgenden drei getrennt voneinander konfektionierten Komponenten enthält:

i) eine erste Komponente (I), die in einem kosmetisch akzeptablen Trägermedium mindestens eine organische Thiol-Verbindung enthält,
ii) eine zweite Komponente (II), die mindestens ein Persulfatsalz enthält, und

iii) eine dritte Komponente (III), die in einem kosmetisch akzeptablen Trägermedium Wasserstoffperoxid enthält,

wobei das Verfahren **dadurch gekennzeichnet ist, dass**

a) in einem ersten Schritt die erste Komponente (I) des Kits, enthaltend in einem kosmetisch akzeptablen Trägermedium mindestens eine organische Thiol-Verbindung, auf die trockenen oder feuchten Fasern aufgetragen wird und für eine Einwirkzeit von 5 bis 45 min auf den Fasern belassen wird,
b) in einem zweiten Schritt die keratinischen Fasern mit Wasser oder einem tensidhaltigen Reinigungsmittel gespült werden,
c) in einem weiteren Schritt eine Mischung aus den Komponenten (II) und (III) des Kits auf die Fasern aufgetragen wird und für eine Einwirkzeit von 10 bis 45 min auf den Fasern belassen wird,
d) und anschließend die Fasern mit Wasser gespült werden oder einem tensidhaltigen Reinigungsmittel ausgewaschen werden.

2. Verfahren nach Anspruch 1, **dadurch gekennzeichnet, dass** die organische Thiol-Verbindung der Komponente (I) ausgewählt ist aus der Gruppe, die gebildet wird aus Cystein, Thioglykolsäure, Thiomilchsäure und/oder deren physiologisch verträglichen Salzen.

3. Verfahren nach einem der Ansprüche 1 oder 2, **dadurch gekennzeichnet, dass** die Komponente (I) einen pH-Wert von pH 2 bis pH 6 oder einen pH-Wert von pH 7,5 bis pH 9,8 besitzt.

4. Verfahren nach einem der Ansprüche 1 bis 3, **dadurch gekennzeichnet, dass** die Komponente (II) mindestens Kaliumperoxodisulfat und/oder Ammoniumperoxodisulfat und/oder Natriumperoxodisulfat und/oder mindestens eine Kombination aus Ammoniumperoxodisulfat und Natriumperoxodisulfat und/oder eine Kombination aus Ammoniumperoxodisulfat und Kaliumperoxodisulfat enthält.

5. Verfahren nach einem der Ansprüche 1 bis 4, **dadurch gekennzeichnet, dass** die Mehrkomponentenverpackungseinheit zusätzlich

iv) eine vierte Komponente (IV), enthaltend in einem kosmetisch akzeptablen Trägermedium mindestens ein Alkalisierungsmittel, ausgewählt aus der Gruppe, die gebildet wird aus Ammoniak, Monoethanolamin, 2-Amino-2-methylpropanol, Triethanolamin, Natriumhydroxid und Kaliumhydroxid, enthält, die mit den Komponenten (II) und (III) des Kits vermischt und auf die Fasern aufgetragen wird und für eine Einwirkzeit von 10 bis 45 min auf den Fasern belassen wird.

6. Verfahren nach Anspruch 5, **dadurch gekennzeichnet, dass** die vierte Komponente (IV) zusätzlich mindestens ein anionisches, nichtionisches, zwitterionisches und/oder amphoteres Tensid enthält.

**Claims**

1. A method for lightening keratin fibers, in particular human hair, which method is **characterized in that** the components of a multicomponent packaging unit (kit of parts) for lightening keratin fibers are applied to the keratin-containing fibers and left on the fibers for from 5 to 60 minutes, and the fibers are then rinsed with water or washed with a surfactant-containing cleansing agent,
the multicomponent packaging unit containing at least the following three components that are packaged separately from one another:

i) a first component (I) which contains, in a cosmetically acceptable carrier, at least one organic thiol compound,
ii) a second component (II) which contains at least one persulfate salt, and
iii) a third component (III) which contains, in a cosmetically acceptable carrier, hydrogen peroxide,

the method being **characterized in that**,

a) in a first step, the first component (I) of the kit, containing, in a cosmetically acceptable carrier, at least one organic thiol compound, is applied to the dry or damp fibers and is left on the fibers for a reaction time of from 5 to 45 minutes,
b) in a second step, the keratin fibers are rinsed with water or a surfactant-containing cleansing agent,

c) in a further step, a mixture of components (II) and (III) of the kit is applied to the fibers and left on the fibers for a reaction time of from 10 to 45 minutes,

d) and the fibers are then rinsed with water or washed with a surfactant-containing cleansing agent.

**2.** The method according to claim 1, **characterized in that** the organic thiol compound of component (I) is selected from the group formed by cysteine, thioglycolic acid, thiolactic acid and/or the physiologically acceptable salts thereof.

**3.** The method according to either claim 1 or claim 2, **characterized in that** the component (I) has a pH value of from 2 to 6 or a pH value of from 7.5 to 9.8.

**4.** The method according to one of claims 1 to 3, **characterized in that** the component (II) contains at least potassium peroxodisulfate and/or ammonium peroxodisulfate and/or sodium peroxodisulfate and/or at least a combination of ammonium peroxodisulfate and sodium peroxodisulfate and/or a combination of ammonium peroxodisulfate and potassium peroxodisulfate.

**5.** The method according to one of claims 1 to 4, **characterized in that** the multicomponent packaging unit additionally contains

iv) a fourth component (IV) containing, in a cosmetically acceptable carrier, at least one alkalizing agent selected from the group formed by ammonia, monoethanolamine, 2-amino-2-methylpropanol, triethanolamine, sodium hydroxide and potassium hydroxide, which component is mixed with the components (II) and (III) of the kit and applied to the fibers and left on the fibers for a reaction time of from 10 to 45 minutes.

**6.** The method according to claim 5, **characterized in that** the fourth component (IV) additionally contains at least one anionic, non-ionic, zwitterionic and/or amphoteric surfactant.

**Revendications**

**1.** Procédé d'éclaircissement de fibres de kératine, en particulier de cheveux humains, **caractérisé en ce qu'**on applique les composants d'un kit multicomposant (kit-of-parts) d'éclaircissement de fibres de kératine sur les fibres de kératine, on les laisse sur les fibres de 5 à 60 minutes, puis on rince les fibres à l'eau ou on les lave avec un produit nettoyant contenant des tensioactifs,

où le kit multicomposant contient au moins les trois composants suivants confectionnés séparément les uns des autres :

i) un premier composant (I) qui contient, dans un médium cosmétiquement acceptable, un composé thiol organique,

ii) un deuxième composant (II) qui contient au moins un sel persulfate, et

iii) un troisième composant (III) qui contient, dans un médium cosmétiquement acceptable, du peroxyde d'hydrogène,

le procédé étant **caractérisé en ce que** :

a) dans une première étape, on applique le premier composant (I) du kit, qui contient, dans un médium cosmétiquement acceptable, un composé thiol organique, sur les fibres sèches ou humides, et on les laisse agir sur les fibres de 5 à 45 minutes,

b) dans une deuxième étape, on rince les fibres à l'eau ou avec un produit nettoyant contenant des tensioactifs,

c) dans une troisième étape, on applique un mélange des composants (II) et (III) du kit sur les fibres, et on les laisse agir sur les fibres de 10 à 45 minutes,

d) et enfin on rince les fibres à l'eau ou avec un produit nettoyant contenant des tensioactifs.

**2.** Procédé selon la revendication 1, **caractérisé en ce que** le composé thiol organique du composant (I) est choisi dans le groupe constitué de la cystéine, de l'acide thioglycolique, de l'acide thiolactique et/ou de leurs sels physiologiquement compatibles.

**3.** Procédé selon la revendication 1 ou 2, **caractérisé en ce que** le composant (I) possède un pH entre 2 et 6 ou entre 7,5 et 9,8.

4. Procédé selon une des revendication 1 à 3, **caractérisé en ce que** le composant (II) contient au moins du peroxodisulfate de potassium et/ou du peroxodisulfate d'ammonium et/ou du peroxodisulfate de sodium et/ou au moins une combinaison de peroxodisulfate d'ammonium et de peroxodisulfate de sodium et/ou une combinaison de peroxodisulfate d'ammonium et de peroxodisulfate de potassium.

5. Procédé selon une des revendications 1 à 4, **caractérisé en ce que** le kit multicomposant contient en plus :

iv) un quatrième composant (IV) contenant, dans un médium cosmétiquement acceptable, au moins un produit d'alcalinisation choisi dans le groupe constitué de l'ammoniac, de la monoéthanolamine, du 2-amino-2-méthylpropanol, de la triéthanolamine, de l'hydroxyde de sodium et de l'hydroxyde de potassium, qu'on mélange avec les composants (II) et (III) du kit et qu'on applique sur les fibres, et qu'on laisse agir sur les fibres de 10 à 45 minutes.

6. Procédé selon la revendication 5, **caractérisé en ce que** le quatrième composant (IV) contient en plus au moins un tensioactif anionique, non-ionique, zwitterionique et/ou amphotère.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1617829
- US 2201929 A
- FR 2719472 A1